(19) **Europäisches Patentamt / European Patent Office / Office européen des brevets**

(11) **EP 3 048 972 B1**

(12) **EUROPEAN PATENT SPECIFICATION**

(45) Date of publication and mention
of the grant of the patent:
**09.11.2022 Bulletin 2022/45**

(21) Application number: **14771542.9**

(22) Date of filing: **17.09.2014**

(51) International Patent Classification (IPC):
**A61B 5/113** *(2006.01)*    **A61B 5/00** *(2006.01)*
**A61B 5/11** *(2006.01)*

(52) Cooperative Patent Classification (CPC):
**A61B 5/7278; A61B 5/113; A61B 5/7221;
A61B 5/7246;** A61B 5/1102; A61B 5/6823

(86) International application number:
**PCT/EP2014/069768**

(87) International publication number:
**WO 2015/044010 (02.04.2015 Gazette 2015/13)**

(54) **PROCESSING APPARATUS, PROCESSING METHOD AND SYSTEM FOR PROCESSING A PHYSIOLOGICAL SIGNAL**

VERARBEITUNGSVORRICHTUNG, VERARBEITUNGSVERFAHREN UND SYSTEM ZUR VERARBEITUNG EINES PHYSIOLOGISCHEN SIGNALS

APPAREIL DE TRAITEMENT, PROCÉDÉ ET SYSTÈME DE TRAITEMENT POUR TRAITER UN SIGNAL PHYSIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2013 EP 13186423**

(43) Date of publication of application:
**03.08.2016 Bulletin 2016/31**

(73) Proprietor: **Koninklijke Philips N.V.
5656 AG Eindhoven (NL)**

(72) Inventors:
• **DERKX, Rene Martinus Maria
5656 AE Eindhoven (NL)**

• **JANSE, Cornelis Pieter
5656 AE Eindhoven (NL)**

(74) Representative: **Philips Intellectual Property & Standards
High Tech Campus 52
5656 AG Eindhoven (NL)**

(56) References cited:
EP-A2- 0 526 973    WO-A1-00/21438
US-A1- 2005 222 510    US-A1- 2006 224 074
US-A1- 2010 312 075    US-A1- 2013 137 936

Processed by Luminess, 75001 PARIS (FR)

**Description**

FIELD OF THE INVENTION

The present invention relates to the field of determining vital signs of a subject, in particular, to a processing apparatus, processing method and system for processing a physiological signal.

BACKGROUND OF THE INVENTION

Vital signs of a subject are powerful indicators in determining a medical condition and fitness of a subject. Vital signs include but are not limited to a respiratory rate (RR) and a heart rate (HR) or pulse rate. The vital signs are descriptive of an underlying physiological process such as heart-beats or a respiratory movement. A physiological signal descriptive of the underlying physiological process can be measured and evaluated.

For example, respiration monitoring can be based on different principles: The measurement of respiratory effort, for example, thorax impedance plethysmography, respiratory inductance plethysmography, often referred to as respiration band or respiband, photoplethysmography or the measurement of respiratory effect, for example, sound recording, temperature sensing, carbon dioxide sensing.

Exemplary sensors for measuring the physiological signal for determining the heart rate comprise a photoplethysmograph sensor, an ECG (electrocardiography) sensor, or a heart sound sensor.

US 2004/0039420 A1 further discloses a pulse detection apparatus that uses physiological signal data obtained from an accelerometer placed on a patient's body to detect the presence of a cardiac pulse. The accelerometer is adapted to sense movement due to a cardiac pulse and produce accelerometer signal data in response thereto as the physiological signal. Processing circuitry analyzes the accelerometer signal data for a feature indicative of a cardiac pulse and determines whether a cardiac pulse is present in the patient based on the feature. The apparatus can be used in a defibrillator for delivering defibrillation pulses to a patient to stimulate a cardiac activity.

The feature indicative of a cardiac pulse can be a temporal energy feature, for example, a peak-to-peak amplitude, a peak-peak amplitude of a derivative, or an energy of the accelerometer signal. Alternatively, the feature can be a spectral energy feature such as the energy or frequency of a peak in the energy spectrum of the signal. In yet another aspect, the feature may be obtained by comparing the accelerometer signal data with a previously-identified pattern known to predict the presence of a cardiac pulse. This is also referred to as pattern matching, wherein the measured physiological signal is compared with a known pattern.

US 2006/0224074 A1 discloses a heartbeat measuring apparatus using pattern maching. Instead of comparing waveform data with a known pattern, a section of waveform data corresponding to one heartbeat is used as a template. A waveform similarity such as correlation between said template and the measured waveform is calculated and used for subsequent analysis.

US 2010/0312075 A1 further discloses signal processing techniques for aiding the interpretation of respiration signals. The peaks of a signal can be rescaled to yield a normalized waveform.

US 2005/222510 A1 discloses methods and apparatus for determining T-wave alternan signatures (i.e., morphology and polarity) derived from a physiologic signal representative of a subject's heart activity.

Since the vital signs are commonly derived from such characteristic features of the physiological signal, there is a need to determine whether the features, for example signal peaks, have been identified correctly. Current approaches to classify whether the peak-detections were done correctly evaluate, e.g., a heart-rate variability or perform a spectral analysis. A prerequisite for applying these techniques is an at least weak-stationary signal.

SUMMARY OF THE INVENTION

It is an object of the present invention to provide a processing apparatus, processing method and system for more accurately determining a quality of signal features, in particular signal features of a non-stationary physiological signal. It is a further object of the present invention to provide a processing apparatus, processing method and system for distinguishing different contributions in a physiological signal.

The invention is defined by the independent claims. Preferred embodiments of the invention are defined in the dependent claims. It shall be understood that the claimed method, system, computer program and recording or storage medium have similar and/or identical preferred embodiments as the claimed processing apparatus and as defined in the dependent claims.

Determining a vital sign such as a heart rate or respiratory rate from a physiological signal is far from trivial, in particular if the physiological signal is obtained via an unobtrusive sensor. The measured physiological signal often comprises signal distortions such as drifts, noise or movement artifacts. Furthermore, the measured physiological signal may comprise information about more than one underlying physiological process such as information about cardiac activity and respiration. For example, a constant heart rate and a constant respiration rate show as distinct peaks in a spectrum of the physiological signal. They can be separated from each other by filtering in frequency-domain. Correspondingly, drifts, noise and artifacts can be suppressed by filtering the spectrum.

While these conventional approaches work well in many applications, there are several limitations: Evaluating a physiological signal by spectral analysis assumes an at least weak stationary signal. However, in case of arrhythmias, i.e., variations, of the underlying physiological process or processes, such approaches do not work anymore. Furthermore, information about the curve shape or morphology is lost. For example, a heart rate is extracted from the spectrum but the shape of the physiological signal corresponding to individual heart beats is distorted by the filtering process.

Furthermore, if the spectra of two underlying physiological phenomena overlap, filtering the physiological signal to extract a first signal, corresponding to a first physiological phenomenon, may impact a spectrum of a second signal, corresponding to a second physiological phenomenon. The signal shape or morphology of the first and second signal should be preserved.

In a further aspect, vital signs can be derived from characteristic features of the physiological signal. However, one has to make sure the features are extracted correctly. This can be indicated by a quality metric. Alternatively, the quality metric can be used for classification of the physiological signal as being good or bad. For example, if the signal feature is a signal peak, a classifier output indicates whether peak-detections from a peak-detector contain errors, e.g., false-positives or false-negatives. Current approaches for determining a quality metric based on heart-rate variability or spectral analysis assume an at least weak stationary signal. In case of arrhythmias, i.e., large variations in peak-to-peak distances, such approaches do not work anymore.

Furthermore, the inventors have found that a shape and spectrum of the physiological signal largely depends on the signal source from which the physiological signal is obtained. For example, a cardiac pulse in an ECG signal differs considerably from new measurement techniques, for example wherein an accelerometer is used for measuring a physiological signal. In particular with new measurement techniques, the curve shape corresponding to a specific physiological phenomenon such as a cardiac pulse can vary significantly from subject to subject.

Furthermore, the waveform of a physiological signal corresponding to a physiologic phenomenon can change depending on the posture of the subject. Both the curve shape and amplitude can be affected. Thus, a plurality of different waveforms can represent one and the same physiological phenomenon which renders a generic pattern comparison difficult.

However, the inventors have found that despite the variation between patients and postures, for one patient, in a given posture, the waveforms corresponding to a physiological phenomenon are very similar. This also holds true for the case of arrhythmias.

Therefore, according to the present invention, a processing apparatus is proposed that takes the physiological signal as well as a feature signal descriptive of occurrences of a feature in the physiological signal as inputs and determines an average waveform around the occurrences. The physiological signal contains at least two wave cycles originating from a physiological process to allow averaging. The average waveform can be invariant to timing and amplitude variations. Based on this average waveform a model signal is determined that comprises amplitude-scaled instances of the average waveform of the physiological signal at occurrences of the feature in the physiological signal. In other words, a copy of the average waveform of the physiological signal around occurrences of the feature in the physiological signal is placed at occurrences of the feature. The amplitudes of each copy of the average waveform can be scaled depending on an amplitude of the physiological signal at or around the occurrence of the feature where the copy is placed. The plurality of copies of the average waveform makes up the model signal.

An advantage of this approach is that it is not necessary that the waveform of physiological phenomena in the physiological signal is known a priori. Furthermore, the processing apparatus can be used for evaluating a non-stationary physiological signal. Thus, also non-periodic physiological signals can be processed. Moreover, the shape of the model signal is not manipulated by frequency-selective filters.

Referring to the system for determining a vital sign of a subject, the processing unit can be configured to determine the vital sign of the subject based on the physiological signal taking into account the model signal. In a first aspect, the model signal is taken into account in that the model signal is used to determine a quality metric of the physiological signal and/or the feature signal. For example, the quality metric can be provided along with the vital sign. In a second aspect, the model signal is taken into account in that the vital sign is determined from the model signal. The model signal in turn is based on the physiological signal. Alternatively, the vital sign is determined based on a difference of physiological signal and model signal as will be described in detail further below.

In a preferred embodiment of the processing apparatus, the physiological signal is a movement signal descriptive of a physiological movement. The processing apparatus can be configured for obtaining a movement signal descriptive of a physiological movement, for example, from an accelerometer or a gyroscope. The proposed processing apparatus is particular advantageous for processing movement signals. A problem of movement signals is that the waveform largely depends on a placement of the sensor and may change significantly depending on the posture of the subject. However, since the processing apparatus according to the present invention determines an average waveform of the physiological signal around the occurrences of the feature in the physiological signal, the shape does not have to be known beforehand.

In a further embodiment, the feature of the physiological signal is a peak of the physiological signal. A peak in this context refers to a local maximum or minimum of the physiological signal. In this embodiment, the feature signal indicates the locations of peaks in the physiological signal. According to the invention, a feature refers to a part of the waveform comprising a predetermined energy, slope, inflection points, local extrema or combinations of the above. In general, any characteristic of the physiological signal can be evaluated as a feature representing an underlying physiological phenomenon.

The resulting model signal can directly be evaluated or used for further processing. For example, the model signal can serve as a "clean" physiological signal. In an alternative embodiment, the processing apparatus is further configured to perform the step of comparing the model signal with the physiological signal.

In an embodiment, the comparing step further comprises the computation of a difference signal between the model signal and the physiological signal. For example, the model signal, corresponding to a first physiological phenomenon, can be removed from the physiological signal which gives a residual physiological signal. An advantage of this embodiment is that the waveform of further contributions to the physiological signal remains untouched. This is in contrast to other filtering techniques (like low-pass filtering, median filtering or Savitsky-Golay filtering) where the residual physiological signal is modified by the filtering process.

In a further refinement, the comparing step further comprises the computation of an energy of the difference signal descriptive of the quality of the feature signal or the physiological signal. The term energy in this context refers to a value descriptive of the difference signal. Optionally, the energy can be normalized by, for example, a value of the physiological signal. For example, the energy represents the value of an integral over a segment of the difference signal. A high energy results from a large difference between physiological signal and model signal and thus represents a large mismatch. A large mismatch can be interpreted as a poor signal quality whereas a small mismatch can be interpreted as a good signal quality. Alternatively, the energy can be compared with a threshold to determine a digital value, for example, indicating 1 if the difference is below a threshold value for high signal quality and 0 if the difference exceeds the threshold. In other words, the energy of the difference signal is inversely proportional to the quality of the feature signal or the physiological signal. The energy value can thus be used for determining a quality metric descriptive of a quality of the feature signal and/or the model signal and/or the physiological signal.

The step of determining the average waveform comprises the step of determining an average relative change between a value of the physiological signal at occurrences of the feature and a value of a neighboring sample of the physiological signal. Thereby, the analysis is independent of predetermined patterns descriptive of physiological features but purely relies on the actually measured physiological signal. For example, at each occurrence of the feature (as specified by the feature signal) the value of the physiological signal is taken and a value of a neighboring sample is subtracted therefrom. The result can be divided by a value of the physiological signal, in particular the value of the physiological signal at the occurrence of the feature or the value of the neighboring sample, to obtain a relative change. For example, for each peak of the physiological signal the value of the sample immediately preceding the respective peak is subtracted and divided by the respective peak value. Thereby, a relative change for this particular neighbor is determined. The relative changes for the neighbors, wherein the neighbors refer to the neighbors immediately preceding the respective peaks, are averaged to determine an average relative change of the immediately preceding sample with respect to a peak.

In other words, the step of determining the average waveform comprises the scaling of the difference between the value of the physiological signal at an occurrence of the feature and the value of a neighboring sample. Thereby the differences for determining the average relative change can be weighted, for example, by the value of the physiological signal at an occurrence of the feature or an inverse thereof. In the latter case, the impact of samples with high values of the average waveform is reduced.

In a further refinement of this embodiment, the step of determining the average waveform comprises repeating the step of determining an average relative change for different neighboring samples to a right and/or a left side, i.e., preceding and succeeding samples, of occurrences of the feature. Thus, not only a relative change for the immediate neighbor is determined but relative changes for samples spaced further apart with respect to the occurrence of the feature to a left and/or right side of the feature are determined and thereby construct the average waveform. In other words, in this embodiment, the average waveform represents the average relative change at predetermined distances from the occurrences of the feature in the physiological signal.

In a further refinement of this embodiment, said repeating is performed until reaching a stop criterion, in particular until a relative change has been determined for all samples of the physiological signal between at least one pair of succeeding occurrences of the features having the smallest temporal separation.

In an embodiment, the step of determining the model signal further comprises scaling an instance of the average waveform depending on a value of the physiological signal at the occurrence of the feature in the physiological signal. Not only the temporal separation of features in the physiological signal can vary, but also the amplitude. Thus, the instance of the average waveform at a particular occurrence of the feature in the physiological signal can be scaled to match an amplitude of the physiological signal. For example, the instance of the average waveform can be scaled such that a value of the average waveform at the occurrence of the feature corresponds to a value of the physiological signal at the occurrence of the feature.

In an embodiment, the step of determining the model signal further comprises the step of interpolating the model signal between instances of the average waveform. As indicated above, there can be a stop criterion for determining the average waveform which is based on the smallest temporal separation of two occurrences of the physiological signal. Thus, there can be a gap between two instances of the average waveform in the model signal which can be filled by interpolating values in-between. Any type of interpolation can be used in particular linear cubic and spline interpolation.

In an embodiment, the processing apparatus is further configured to evaluate a variation of the temporal separation of occurrences of a feature in the feature signal. Advantageously, the proposed determination of a quality metric based on an energy of the difference signal as disclosed herein, can be combined with alternative approaches for determining a quality of the feature signal and/or the physiological signal. However, even if a large variation of the temporal separation is detected, for example due to a non-life threatening arrhythmia, the signal can still be classified as good if the quality metric based on the energy of the difference signal indicates so.

In yet another embodiment, the processing apparatus is further configured to evaluate the physiological signal and/or feature signal in time-domain. Advantageously, blocks, i.e., segments of the physiological signal and corresponding feature signal, of limited length are evaluated. Advantageously, blocks of a duration of 1 to 8 seconds are evaluated. This is an advantage compared to frequency domain processing, wherein typically longer windows of, for example, 30 seconds are evaluated for meaningful results in frequency domain.

BRIEF DESCRIPTION OF THE DRAWINGS

These and other aspects of the invention will be apparent from and elucidated with reference to the embodiment(s) described hereinafter. In the following drawings

Fig. 1 shows an embodiment of device for determining a vital sign of a subject;
Fig. 2 shows a perspective view of the embodiment of the device for determining a vital sign of a subject;
Fig. 3 shows a schematic block diagram of an embodiment of a device for determining a vital sign of a subject;
Fig. 4 shows a graph of respiratory signals for determining a respiratory rate of a subject;
Fig. 5 shows a graph of a heart signal for determining a heart rate of a subject;
Fig. 6 shows an exemplary embodiment of a processing unit;
Fig. 7 shows a flowchart of an embodiment of a processing method;
Fig. 8 shows a flowchart of determining an average waveform and a reference signal;
Figs 9A to 9D show waveforms illustrating different steps of determining the reference signal;
Figs. 10A and 10B show two different graphs of obtained reference signals;
Figs. 11A to 11C show waveforms illustrating different steps of determining a reference signal,
Fig. 12 shows an embodiment of a system for removing an electrocardiogram (ECG) overlay in an electromyogram (EMG) measurement; and
Fig. 13 shows a graph of the corresponding signals.

DETAILED DESCRIPTION OF THE INVENTION

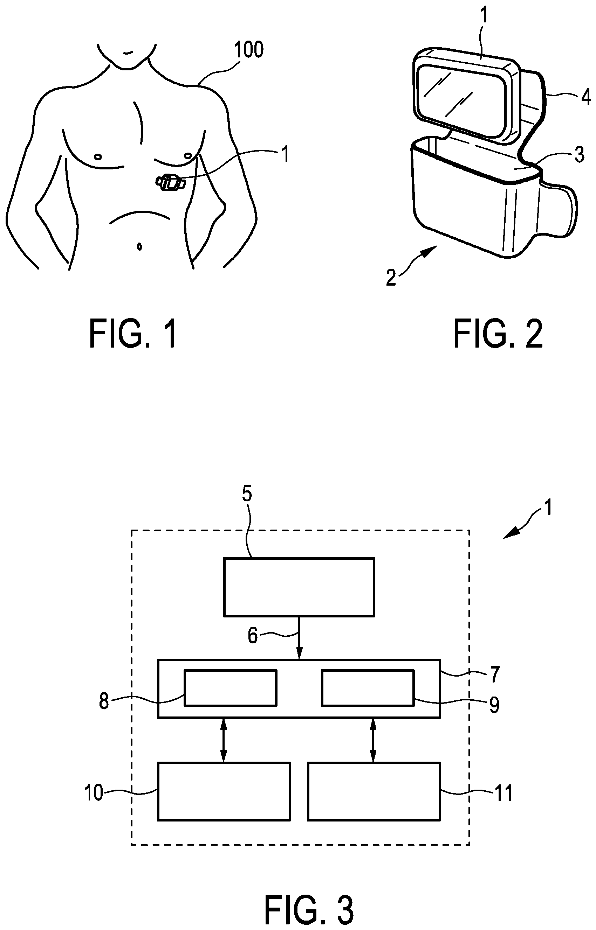

Fig. 1 shows an embodiment of a device 1 for determining a vital sign of a subject 100. The device 1 is applied to a suitable location on a body of the subject 100 for measuring a physiological signal. Suitable locations include but are not limited to the chest and rib-cage region as depicted in Fig. 1. However, any part of the subject 100 that is affected by the physiological phenomenon, in particular the upper torso, can be envisaged.

The device 1 for determining a vital sign of a subject 100 according to an aspect of the present invention enables an unobtrusive measurement of, for example, a movement signal descriptive of a respiratory movement and/or a movement due to cardiac pulses. The signals form the basis for determining a respiratory rate and a heart rate (pulse rate) of the subject. As can be seen in Fig. 1, the device 1 can be implemented as a small device that can simply be applied to the body of the subject 100.

Conventional respiration monitoring devices, for example, a nasal canula are inconvenient for the patient. Alternative conventional respiration monitoring devices such as a chest belt for respiratory inductance plethysmography require more effort in handling. For respiratory inductance plethysmography (RIP) one or two RIP-bands have to be wrapped around the chest and/or abdomen of the subject 100. Especially for a heavy patient, the application of these bands can be cumbersome for a nurse. Nonetheless, respiratory inductance plethysmography is one of the most common techniques used for determining a respiration rate today. Further alternative conventional respiration monitoring devices include devices for thorax impedance plethysmography that are used in intensive care units. In thorax impedance plethysmography, the impedance is measured via two electrodes, which can be inconvenient for the patient. Correspondingly, for conventional heart rate measurements either ECG electrodes or a finger clip for photoplethysmography are applied to the patient.

Fig. 2 shows the device 1 for determining a vital sign of a subject 100 and an application pocket 2. The application pocket 2 can be a disposable item that is applied to the skin of the subject 100 as shown in Fig. 1. The application pocket 2 comprises a compartment 3 for receiving the device 1 and a cover lid 4 for closing and fixing the device 1 in the compartment 3. Applying the device 1 to the skin of the subject 100 includes, but is not limited to this embodiment. For example, the device 1 can simply be taped to the skin of the subject 100. Alternatively, the device 1 can also be attached to clothing of the subject 100. In an example, the device 1 can be seated in a pocket of the tight shirt. The device 1 just has to follow the movement of the underlying physiological phenomenon to be measured. In some cases, the measurement may be limited to detecting a respiratory movement.

Fig. 3 shows a block diagram of an exemplary embodiment of the device 1 for determining a vital sign of a subject. The device is an exemplary embodiment of the system for determining a vital sign of a subject wherein as least some of the components are e.g. implemented in a common housing. The device 1 comprises a sensor 5 for measuring a physiological signal 6, a first processing apparatus 8 for determining a feature signal descriptive of occurrences of a feature in the physiological signal 6, and a second processing apparatus 9 for evaluating the physiological signal according to an aspect of the present invention. In this embodiment, the physiological signal 6 is a movement signal descriptive of a physiological movement, in particular a respiratory movement and/or a physiological movement due to cardiac pulses. In this embodiment, the first processing apparatus 8 for determining the feature signal and the second processing apparatus 9 for evaluating the physiological signal are implemented as a common processing unit 7. Alternatively, they can be implemented separately. In other words, the functional elements of the processing unit 7 can be implemented as distributed sub-units. The implementation of the respective functionality can be in hardware or software, for example software executed by a microprocessor. In this exemplary embodiment, the device 1 further comprises an interface 10 and a memory 11.

The sensor 5 may, for example, comprise an accelerometer which is adapted to generate a movement signal indicative of the acceleration along one or more spatial axes. In this embodiment, the accelerometer is a tri-axial accelerometer adapted to generate a movement signal that comprises three accelerometer signals indicative of the acceleration along three orthogonal spatial axes. For example, three-axial accelerometers named Bosch BMA355, ST Microelectronics LIS3DSH, ST Microelectronics LIS344ALH or Kionix KXM52 can be used. Alternatively, the sensor 5 may comprise a gyroscope which is adapted to generate a movement signal indicative of an angular rotation. For example, the gyroscope ST Microelectronics LYPR540AH can be used to acquire angular rates for three orthogonal axes from which angular rotations can easily be derived.

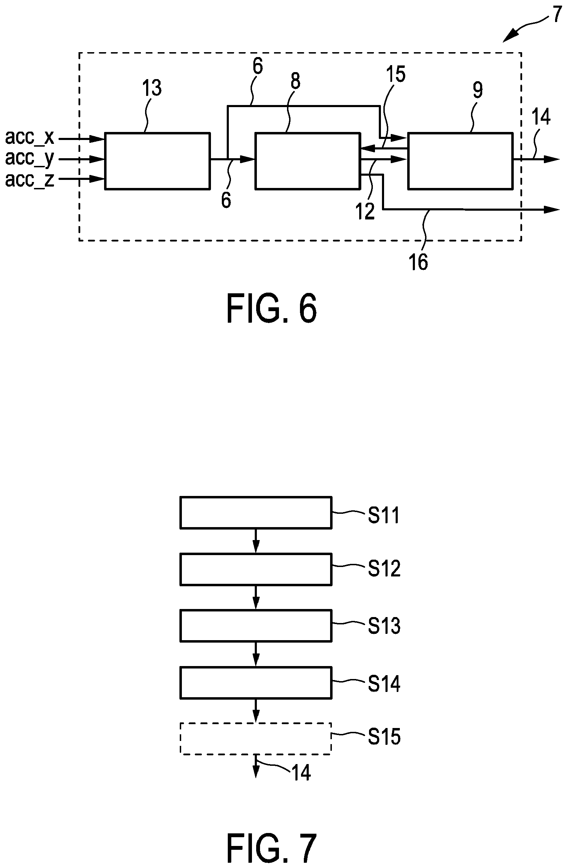

The movement signal 6 is provided to the processing unit 7 for determining a vital sign of the subject. An exemplary embodiment of a processing unit 7 will be described further below with reference to Fig. 6.

The interface 10 can be a wireless interface for providing a vital sign signal of the subject. Advantageously, the device 1 is battery powered device and the interface 10 is a wireless interface such that no cables are required. For example, the interface 10 can provide the vital sign to a patient monitor for displaying the vital sign on a screen. Alternatively, the interface 10 can be a human machine interface (HMI) for showing the vital sign and/or the physiological signal and/or any quantity derived thereof on a display of the device 1.

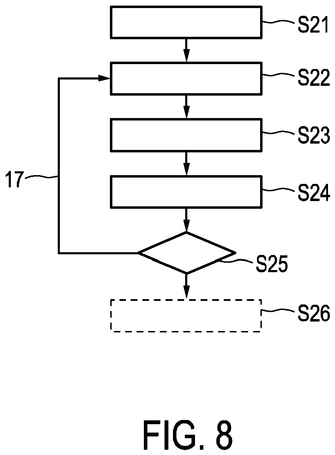

The memory 11 can store the physiological signal and/or the feature signal derived therefrom and/or the determined vital signs that have been determined by the processing unit 7. Alternatively, the memory 11 can be a non-transitory storage medium containing instructions for execution by the processing unit 7 or components thereof, wherein the instructions cause the processing unit 7 to perform at least some of the steps of the flowchart shown in Fig. 7 and/or Fig. 8. If the memory 11 stores the determined physiological signal 6, the signal can be recorded over a period of time, such that no connection to an external entity such as a patient monitor is required. The physiological signal can be downloaded after a desired measurement period. Thereby, the power consumption can be further reduced since no communication, in particular no wireless communication, is required.

In an alternative embodiment, the sensor 5 and the processing unit 7 or parts thereof are not implemented in one device. For example, a minimum configuration of sensor 5 and interface 10 can be implemented as a device 1 that is worn by the patient. The physiological signal 6 is thus transmitted via the interface 10 to a processing unit 7 at a remote location. For example, the processing unit 7 can be implemented as a part of a patient monitor or part of a health care infrastructure such as a hospital IT system. In a further embodiment, a smartphone or similar device serves as the processing unit 7 and is configured to perform the steps for determining a vital sign of the subject and/or to perform the steps for processing a physiological signal according to an aspect of the present invention.

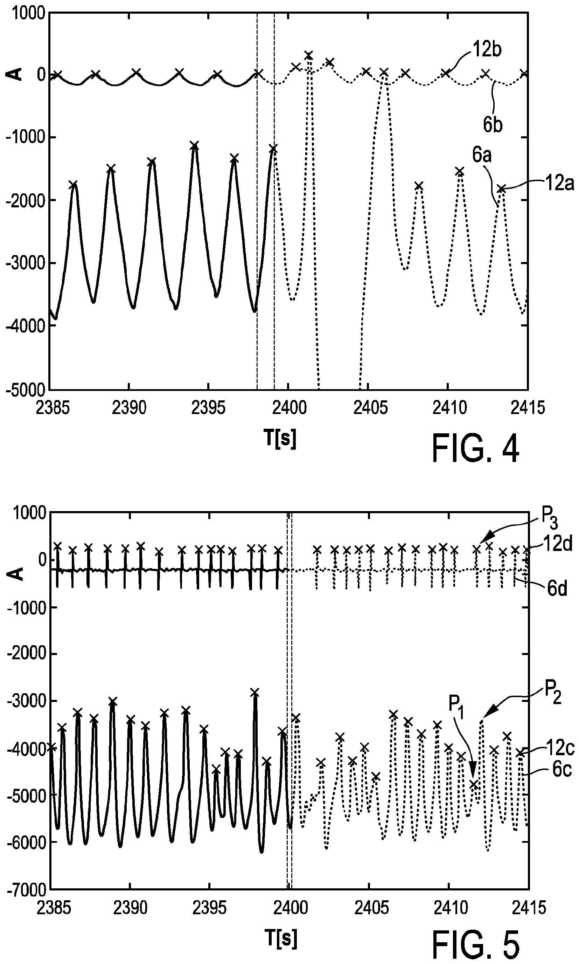

Fig. 4 shows a graph of two physiological signals 6a, 6b and two feature signals 12a, 12b. The horizontal axis denotes the time T in seconds, whereas the vertical axis represents the amplitude of the respective signals. The lower curve in Fig. 4 shows a physiological signal 6a being a movement signal descriptive of a physiological movement due to respiration that can be obtained via an accelerometer. In this embodiment, a tri-axial accelerometer is used and pre-processing applied to the raw signals of the accelerometer to obtain the physiological signal 6a. The feature signal 12a, indicated by crosses, describes occurrences of peaks of the physiological signal 6a.

The second physiological signal 6b has been obtained using an alternative measurement technique, i.e. respiratory inductance plethysmography, which is also often referred to as respiration band or respiband. The second feature signal 12b, indicated by crosses, denotes peaks of the second physiological signal 6b. It should be noted that the respiband physiological signal 6b shows a very clean signal. Thus, the features of the feature signal 12b can be derived from the physiological signal 6b with high accuracy and the feature signal 6b has an inherently high quality. This respiband physiological signal 6b serves for comparison and for evaluating the performance of the accelerometer-based physiological signal 6a.

The dashed vertical lines in Fig. 4 denote a delay, wherein the physiological signal 6a is delayed with respect to the physiological signal 6b. This is due to the experimental setup. In a segment to the left of the vertical dashed lines, the accelerometer-based physiological signal 6a shows good correspondence with the respiband physiological signal 6b in the upper part of Fig. 4. In a segment to the right of the vertical dashed lines, a movement artifact is visible after time T = 2400 seconds. The respiband physiological signal 6b only shows a slight disturbance and still enables an extraction of the peaks. However, the movement artifact substantially disturbs the physiological signal 6a obtained with the accelerometer.

Fig. 5 refers to heart monitoring. The horizontal axis denotes the time T in seconds, whereas the vertical axis represents the amplitude of the respective signals. The lower curve in Fig. 5 denotes a physiological signal 6c representing a physiological movement due to cardiac pulses. Also in this non-limiting example, the feature signal 12c describes occurrences of peaks in the physiological signal 6c. For comparison, the upper curve in Fig. 5 shows a physiological signal 6d obtained with ECG electrodes, thus an electrocardiogram. Also in this example, the upper curve is very clean signal, wherein the occurrences of peaks can be clearly identified as shown by the feature signal 12d.

Both physiological signals 6c and 6d are obtained from the same subject. The time delay between the two signals is indicated by vertical dashed lines, wherein the accelerometer-based physiological signal 6c is slightly delayed with respect to the ECG-based physiological signal 6d.

It should be noted that the subject in this example has a non-steady, irregular heart rate that shows arrhythmias. Just after second 2400 and after second 2410, there are wide intervals between two heart beats (inter-beat-interval, IBI). In this example, a classifier or quality metric that is solely based on the peak-to-peak variances, i.e. a classifier that evaluates how the separation of neighboring peaks fluctuates may classify the signal as being bad. Furthermore, a classifier that evaluates spectral features may fail and classify the signal as being bad because arrhythmias cause distortions to the spectrum of the measured physiological signal. For example, multiple peaks may be present in the spectrum or the spectrum around a peak may be significantly broadened.

A classifier is usually determined for segments of the physiological signal. If the classifier indicates a poor signal quality, the entire segment is discarded and not evaluated. As a consequence a reduced number of segments of the physiological signal is evaluated and the coverage of the physiological signal is lowered.

In Figs. 4 and 5, the segments of the physiological signals 6a, 6c to the left of the vertical dashed lines are of high quality, whereas the segments to the right of the vertical dashed lines show distortions. For example, in Fig. 5, lower curve 6c, the feature signal erroneously contains the peak denoted by $P_1$ as a feature corresponding to a physiological phenomenon, whereas the following peak denoted by $P_2$ is discarded. For example, the peak denoted by $P_2$ is discarded because the technique for obtaining the feature signal requires a spacing to the previous identified peak $P_1$ above a predetermined threshold. However, a comparison of the accelerometer-based physiological signal 6c with the ECG-based physiological signal 6d shows that the peak at $P_1$ should have been discarded whereas the peak $P_2$ corresponds to an actual cardiac pulse denoted by $P_3$.

Fig. 6 illustrates a processing unit 7 according to an exemplary embodiment in more detail. The processing unit 7 comprises a pre-processing apparatus 13 for generating a physiological signal based on sensor signals, a first processing apparatus 8 for determining a feature signal descriptive of occurrences of a feature in the physiological signal, and a second processing apparatus 9 for evaluating a non-stationary physiological signal.

In this embodiment, the pre-processing apparatus 13 receives the output signals acc x, acc_y, acc_z of a tri-axial accelerometer and combines these signals into a physiological signal 6 that is provided to the first processing apparatus 8 and the second processing apparatus 9. The pre-processing apparatus 13 is an optional element that can be part of a sensor 5 (as shown in Fig. 3) or part of a processing unit 7. In an embodiment, the pre-processing consists of resampling the sensor signal, filtering of the three accelerometer axes (acc x, acc_y and acc z) and/or a fusion of the accelerometer axis into one physiological signal 6. Alternatively, an output signal of a sensor, e.g. an accelerometer or PPG sensor, directly serves as the physiological signal.

In the embodiment described herein, the first processing apparatus 8 for determining a feature signal descriptive of occurrences of the feature in the physiological signal 6 is a peak-detector. However, it should be noted that the invention is not limited in this respect and that other features of the signal such as extrema of the wave, extrema of a gradient of the wave, i.e. a slope of the signal, inflection points, a peak energy and the like can also be considered as features. The peak-detector (first processing apparatus 8) provides a feature signal 12 to the second processing apparatus 9. The features signal 12 is descriptive of occurrences of peaks in the physiological signal. However, the peak-detection is not necessarily perfect. Sometimes a peak is missed (FN: false negative) and sometimes peaks are wrongly detected (FP: false positive). Thus, the second processing apparatus 9 in this embodiment is configured to classify whether the peak-detection is accurate.

The second processing apparatus 9 in this embodiment can also be referred to as a classifier unit. In an embodiment, the classifier unit computes a quality metric descriptive of a quality of the feature signal 12 based on a window, i.e. a section, of the physiological signal 6 of, for example 8 seconds length, and classifies the physiological signal 6 and/or the feature signal 12 as either being good or bad. The classifier result is provided at an output 14 of the classifier unit 9.

Optionally, the classifier unit 9 also provides a feedback path 15 to the peak-detector 8. Thereby, the peak-detector 8 receives information whether a segment of the physiological signal 6 is good and should be used for determining a vital sign signal 16 or whether this particular segment should be discarded and not used for determining a vital sign of a subject. In this exemplary embodiment, the vital sign of the subject is a heart rate. However, the invention is not limited in this respect and could also be used for different physiological signals and vital signs, for example, for processing a respiratory signal as the physiological signal 6 and for determining a respiratory rate as the vital sign.

The processing of a physiological signal 6 by the processing apparatus 9 is described in more detail with reference to the flowchart shown in Fig. 7.

Fig. 7 shows a flowchart of the steps performed by the processing apparatus 9 according to an aspect of the present invention. In a first step S11 a physiological signal is obtained. In a preferred embodiment, the physiological signal is a movement signal descriptive of a physiological movement, i.e. a movement caused by a physiological phenomenon. The physiological signal can be an output of a corresponding sensor or can also refer to a pre-processed signal or a previously recorded signal that is evaluated at a later point in time.

In a second step S12, a feature signal descriptive of occurrences of a feature in the physiological signal is obtained. For example, the feature signal can indicate the time or locations such as the samples of the physiological signal, where a feature such as a peak occurs. For example, the feature signal can be obtained from a peak-detector 8 as shown in Fig. 6. Alternatively, the processing apparatus for processing the physiological signal is further configured to determine a feature signal descriptive of occurrences of a feature in the physiological signal.

In a third step S13 an average waveform of the physiological signal around said occurrences of the feature in the physiological signal is determined. For example, the peaks of the physiological signal are taken as a starting point and for each peak, the difference of its neighboring values with respect to the value of the peak are determined. Based on these differences, for each distance with respect to a peak, an average difference is determined. The difference between a peak and neighboring value is weighted, for example, weighted by the height of the corresponding peak or the inverse thereof to obtain a relative change.

In a next step S14, a model signal comprising instances of the average waveform of the physiological signal at the occurrences of the feature in the physiological signal is determined. In other words, for each peak a copy of the average waveform is instantiated and arranged such that the peak of the average waveform corresponds to the respective peak of the physiological signal. The height of the average waveform is scaled by the height of the respective peak such that the model signal comprises amplitude-scaled instances of the average waveform.

In a last optional step S15, a quality metric descriptive of a quality of the feature signal is determined based on a comparison of the physiological signal and the model signal. For example, a difference signal between the physiological signal and the model signal is computed. Optionally, the difference signal is normalized, for example by peak values or an average value of the physiological signal. An energy of the difference signal is computed which is descriptive of the quality of the feature signal and/or the physiological signal. A small difference and thus small energy of the difference signal indicates a good matching and high signal quality whereas a high difference indicates poor matching and low signal quality. The output 14 of this step is a classifier output based on the quality metric. This quality metric is computed as, for example directly an energy value or normalized energy value of the difference signal. Alternatively, the computed difference is compared with a threshold to determine a digital output, for example 1 if the signal quality is good and the respective segment of the physiological signal should be used for determining a vital sign, or 0 which means that the segment should be discarded. In an embodiment, a classifier determines whether a particular excerpt belongs to a specific, distinct class. The output of the classifier can be a label or identifier of one of these classes

It should be noted that the steps of determining an average waveform of the physiological signal around occurrences of the feature in the physiological signal S13 and a step S14 of determining a reference signal can also be performed in a combined step or also in an iterative manner. For example, not the entire average waveform is determined before determining the model signal. Instead, the average waveform and the model signal can also be determined step by step on a sample-by-sample basis.

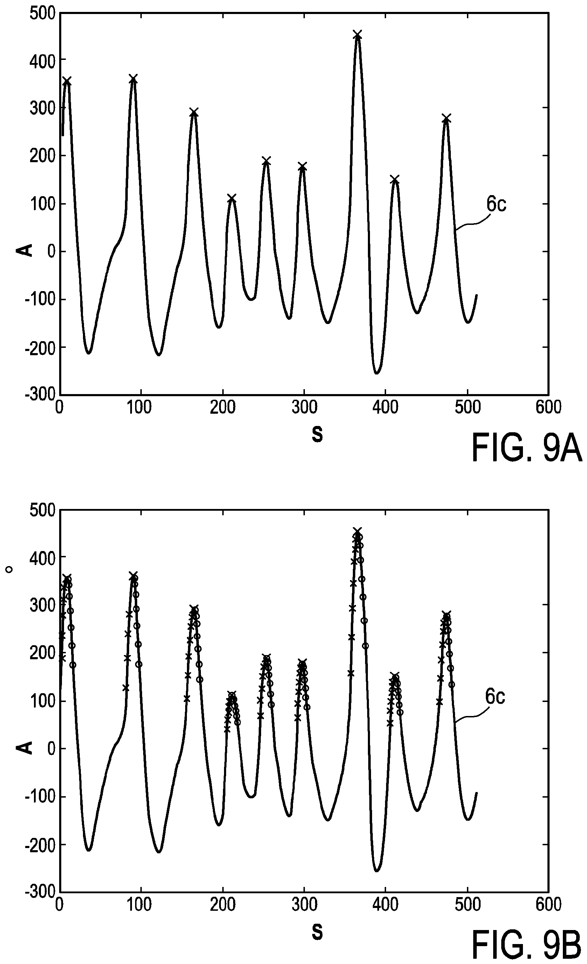

An exemplary embodiment of determining a model signal based on averaging the physiological signal around occurrences of the feature in the physiological signal is illustrated with reference to Fig. 8. Intermediate steps of how the model signal can built up are shown in Figs. 9A to 9D.

Referring to Fig. 8, in a first step S21, the model signal is initialized. In the given example, the physiological signal is processed on a block-by-block basis of nonoverlapping segments of the physiological signal. In this embodiment, the physiological signal has a sample rate of 62.5 Hz and each block comprises 512 samples, Fig. 9A to 9D. However, the invention is not limited in this respect. This segment has a duration of about 8 seconds. At the occurrences of the peaks, the reference signal is initialized with the values of the physiological signal of the peaks. This is illustrated in Fig. 9A. The physiological signal 6c in Figs. 9A to 9D corresponds to the physiological signal 6c in Fig. 5 wherein the segment from second 3292 to 2400 is depicted. The horizontal axis denotes the sample number S, whereas the vertical axis represents the amplitude A of the respective signals. The segment shows a clear arrhythmia of the heart beats, i.e. a varying spacing or inter-beat-interval (IBI) of the peaks. The model can be initialized with peak values, as identified by the peak-detector, by computing a vector m_est(p) = peak_value(p). Optionally also the inverse-peak-values are determined to save computational complexity. Assuming that there are P peak-values, P inverse peak-values have to be computed which results in an auxiliary vector peak_value_inverse(p) = 1/peak_value(p).

In a second step S22, for every neighbor value of the peaks, a relative change with respect to the peak value is determined by calculating change(p) = (peak_value(p) - neighbor_value(p)) * peak_value_inverse(p), where p indicates a peak-number index p = [0..P-1]. A neighbor value as used herein refers to a sample that is located at a left or right side with respect to a peak. For example, the first neighbor to the left is the first sample to the left in Fig. 9B, with respect to the location of an occurrence of a peak. In other words, each peak can have a first left neighbor (provided that the peak is not the first sample of the segment, however, this is not the case in the example illustrated in Figs. 9A to 9D).

In step S23, an average relative change of a particular neighbor value is determined for all peaks, for example by calculating Avg_change = sum(change(p))/P. For example, for the first left neighbor for each peak the relative change of the first left neighbor to the respective peak is calculated. Then the sum of all these relative changes is calculated and the result divided by the number of peaks.

In a next step S24, the auxiliary vector m_est(p) is updated based on the value of the peaks peak_value(p) and the determined average relative change Avg_change by calculating m_est(p) = (1-Avg_change(p)) * peak_value(p). The multiplication by the peak value gives an amplitude scaled value of the average relative change. This auxiliary vector is used for updating the reference signal in that, for example, the first left neighbor of the first peak is assigned the value of the first element of the auxiliary vector m_est(p), the first left neighbor of the second peak is assigned the second value of the auxiliary vector m est(p), and so on.

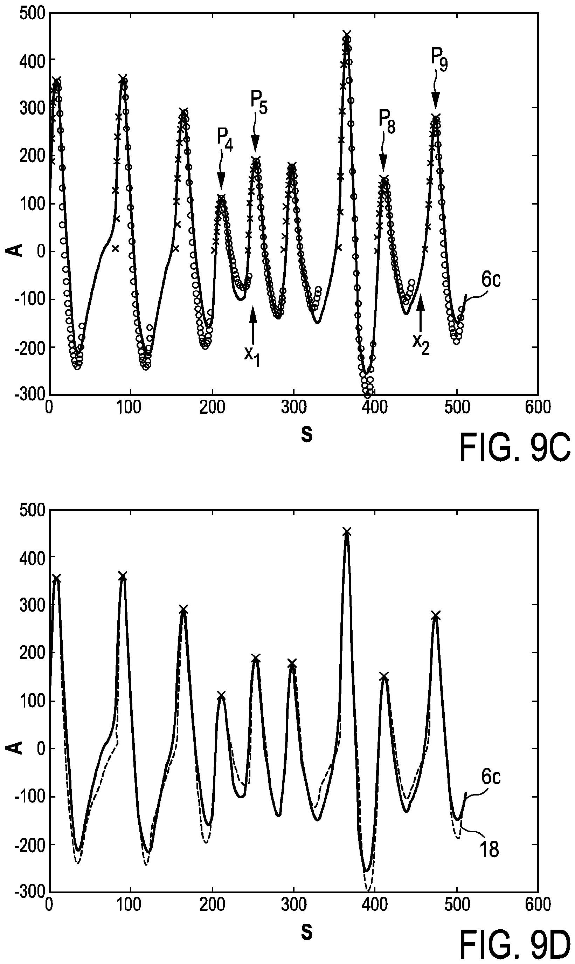

The same calculations can be performed for further left neighbors and further right neighbors. This is indicated by a loop 17 in Fig. 8. The decision step S25 causes a repetition of the steps S22 to S24 for different neighboring values to a left end or right side of the peaks for every next neighbor of the peaks until the indices of two samples belonging to the model signal of two neighboring peaks, for example, the sample number of a left neighbor of the second peak and a sample number of a right neighbor of the first peak, coincide. In Fig. 9C, this stop criterion is fulfilled for the sample indicated by X1 between the peaks P4 and P5, wherein a sample number of a right neighbor of P4 and a sample number of a left neighbor of P5 coincide.

In a last optional step S26, sample values of the reference signal that have not been calculated in the aforementioned process can be interpolated to close gaps. An exemplary gap, indicated by X2, occurs between peaks P8 and P9.

The procedure according to this aspect of the present invention is illustrated in with reference to the graphs shown in Figs. 9A to 9D. The starting point and initialization is illustrated by Fig. 9A. Fig. 9B shows an intermediate graph, wherein the steps S22 to S24 have been repeated seven times on each side, i.e. for the seven neighbors to a left side of each peak and the seven neighbors of a right side of each peak. In this intermediate graph, the reference signal extends towards the left side (shown as crosses) and the right side (shown as circles) of the initial peak detections. It should be noted that the number of samples to a left side can be different from a number of samples to a right side of a peak. For example, an earlier stopping criterion can be implemented for the left side as exemplarily shown with reference to Fig. 9C. For example, the stopping criterion can stop adding further neighboring samples to a left side of a peak when the values of the reference signal are below a threshold such as a baseline at 0. Fig. 9C shows that the reference signal is further grown to a right side of the peaks, until the indices of a left neighbor of a peak and a right neighbor of another peak coincide. This is indicated with X1, wherein the indices (but not necessarily the values) of a right neighbor of peak P4 and a left neighbor of peak P5 coincide.

Finally, as described with reference to step S26 in Fig. 8, gaps of the model signal are closed by interpolating the model signal to arrive at the final model signal. In this step, values of the reference signal are determined that have not been calculated in steps S22 to S24 during the repetitions, for example, from sample 50 to 80 and 110 to 150, because these are heart cycles with a long peak-to-peak interval. The final reference signal 18 is shown together with the physiological signal 6c in Fig. 9D.

Optionally, a classifier output is computed based on a quality metric. This quality metric is descriptive of a quality of the feature signal, calculated based on a comparison of the physiological signal and the reference signal. The quality metric can be computed as an energy of a difference signal between the model signal 18 and the physiological signal 6c.To evaluate how well the model signal matches the physiological signal, the quality metric can be computed, for example, by energy=sum(abs(physiological_signal - model_signal))/sum(abs(physiological_signal)). If the model signal is not computed for all samples of the physiological signal, the computation of the quality metric or energy can be limited to those samples where a model signal is available. The normalization by the absolute values of the physiological signal is optional but can help in comparing segments with different signal magnitude. For the example shown in Fig. 9D, the quality metric value is 0.2056.

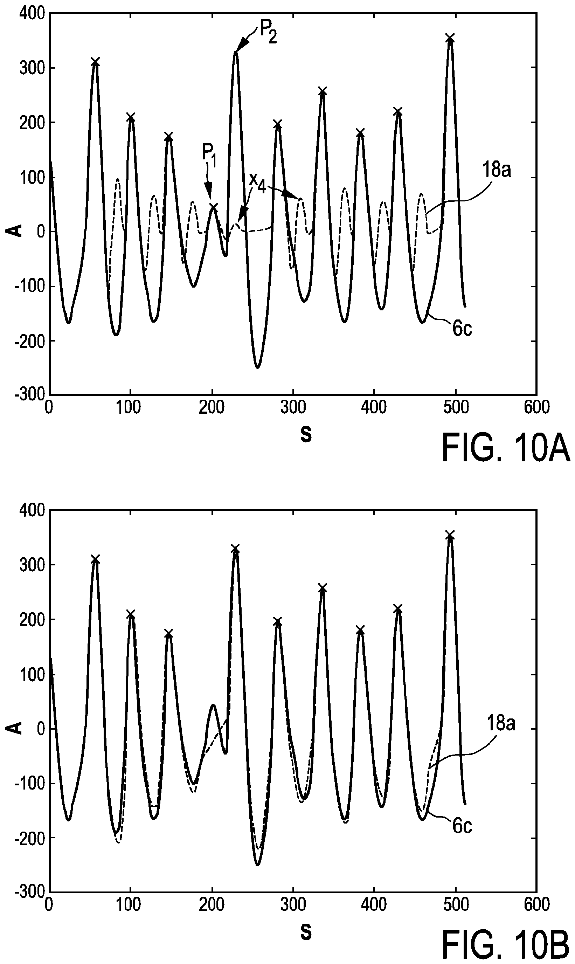

Figs. 10A and 10B illustrate the determining of a quality metric for the segment of the physiological signal 6c of Fig. 5 from seconds 2408 to 2416, i.e. for the segment with the wrong peak-detection, wherein peak P1 has been detected instead of peak P2.

Fig. 10A illustrates the physiological signal 6c and the determined model signal 18a, wherein the model signal is determined based on a feature signal, wherein the peak P1 is erroneously considered and peak P2 has been discarded. For this case the quality metric is 0.6357. Clearly, the model signal and thus the determined quality metric is heavily influenced by the missed peak-detection P2. The missed peak-detection causes the bump in the average waveform that is indicated by X4 in the model signal.

The situation for a correct detection, i.e. wherein the peak $P_2$ at sample-index 230, instead of peak $P_1$ at sample-index 200, is detected, is shown in Fig. 10B. The resulting model signal is indicated by curve 18b. In this correct case, the quality metric is 0.1674 compared to 0.6357 in the wrong case shown in Fig. 10A.

It should be noted that in case of large movement artifacts, the value of the quality metric or energy of the difference signal can exhibit even more extreme values, for example 10 or 100. However, the value of the quality metric is not limited in this respect and depends on the desired normalization scaling and amplitude of the evaluated physiological and reference signal.

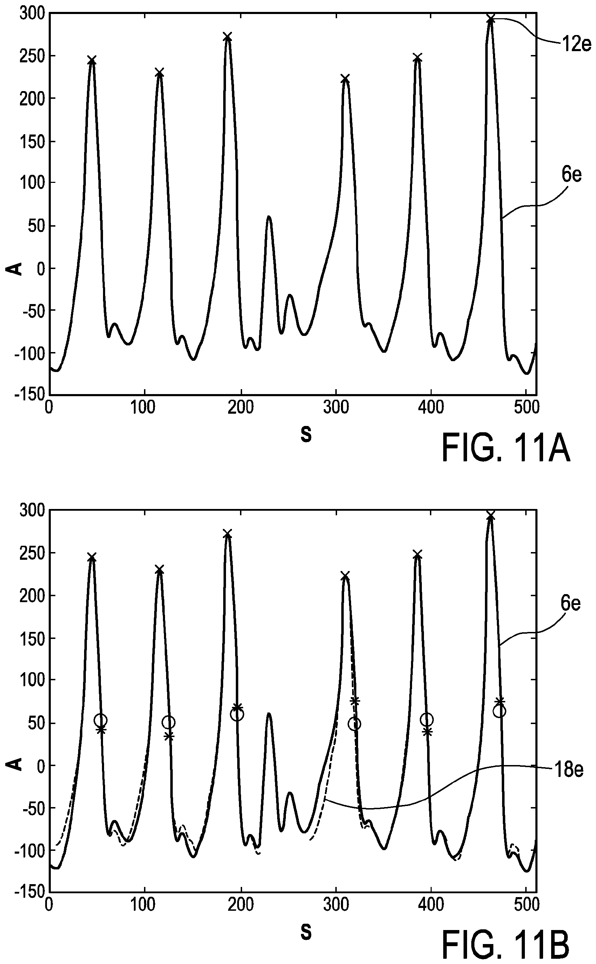

A more detailed example of how a quality metric can be determined including details of an exemplary calculation of average waveform and model signal is described with reference to Figs. 11A to 11C.

The proposed signal processing is based on the finding that each wave cycle in the physiological signal has a similar shape, however, not necessarily a certain (i.e. regular) temporal separation and/or amplitude of the wave cycles. For example, amplitudes of a heart-wave can depend on the breathing.

The basic steps of determining the model signal in this example can be summarized as (1) finding the peaks as starting points, (2) for each (left and/or right) neighboring sample: compute the average relative drop/increase in level compared to the initial-peaks, and compute the values of the model signal based on the average drop/increase and initial-peaks, and (3) a (linear) interpolation of further values of the model signal, when any two neighboring samples coincide.

Fig. 11A shows a segment of a physiological signal 6e. The segment or window in this non-limiting example has a duration of 8 seconds and comprises 512 samples. The feature signal 12e shows six clearly defined peaks, whereas the peak at around sample-index 220 is debatable whether this peak should be included or not. The peak-detector may, for example, be adapted not to detect smaller peaks that quickly follow after larger detected peaks. Thus, the peak at sample-index 220 is not included in the feature signal.

For describing the further algorithm hereafter, the physiological signal is denoted as a vector $\underline{x} \hat{=} \underline{p}_P[w_P]$ with $w_P$ being the index of the 8 second window. The length of $\underline{x}$ equals $N_x$. In the example in Fig. 11A, there are $N_x = 512$ samples. The peak-locations of the physiological signal $\underline{x}$ are listed as a vector $\underline{p}$ that has a length of $N_p$. In the example of Fig. 11A, there are 6 detected peaks and hence we have $N_P = 6$ and the vector $\underline{p}$ contains the values: 43, 115, 187, 310, 386 and 462.

The waveform values at the detected peaks-locations are denoted by:

$$\left(\underline{x}_p\right)_j \hat{=} \left(\underline{x}\right)_{(\underline{p})_j},$$

where $j = 0, ..., N_p - 1$ is the index of the peak. In the example of Fig. 11A, the vector $\underline{x}_p$ contains the values: 249, 234, 278, 227, 251 and 296.

The next step in determining the average waveform and determining the model is to look for neighboring samples (toward the left and right with respect to the initial peaks) and to compute the average drop or increase compared to the initial peak-levels.

The neighboring locations with respect to the peak-locations can be defined as:

$$\underline{p}_{\Delta k} = \underline{p} + \Delta_k.$$

where $k = 1$ defines the scanning of neighboring samples to the left, whereas $k = 2$ defines the scanning of neighboring samples to the right, thus

$$\Delta_k < 0 \quad \text{for:} \quad k = 1$$

$$\Delta_k > 0 \quad \text{for:} \quad k = 2.$$

For all neighboring locations with respect to. the peak-locations which are inside the 8 second window, the average level-drop/increase is computed as:

$$\psi \Delta_k = \frac{1}{N} \sum_{j=0}^{N_p - 1} \frac{(\underline{x}_p)_j - (\underline{x})_{[(\underline{p})_j + \Delta_k]}}{(\underline{x}_p)_j}$$

$$0 \leq \left[(\underline{p})_j + \Delta_k\right] < N_x$$

where N equals the number of neighboring locations that are inside the region $[0,...,N_x -1]$ computed as:

$$N = \# \left\{ \forall_j = 0...N_{p-1} : 0 \leq \left[(\underline{p})_j\right] < N_k \right\}.$$

Thereby, the quantity $\psi$ for the neighbors of the respective peaks gives the average waveform of the physiological signal around the occurrences of the peaks. In the given example, the drops/increases are scaled by the values $(\underline{x}_p)_j$ of the peaks.

Fig. 11B illustrates the calculation for the tenth neighbor to the right of each of the peaks, i.e., for $\Delta_2 = 10$. The values of the physiological signal at the tenth neighbors to the right of the peaks are given by 45, 35, 67, 75, 40 and 74. The values are indicated by an asterisk in Fig. 11B. The resulting value for the average waveform is $\psi\Delta_k = 0.7815$.

The model signal, for comparison with the physiological signal $\underline{x}$, is denoted by $\underline{m}$. The length of $\underline{m}$ is equal to the length of $\underline{x}$ and is denoted by $N_m = N_x$.

The values of the model signal are computed as:

$$\forall_{j:0} \leq \left[(\underline{p})_j + \Delta_k\right] < N_x : (\underline{m})_j = (\underline{x}_p)_j \cdot (1 - \psi \Delta_k).$$

In Fig. 11B, the values of the model signal are indicated by a circle at the neighboring samples of the peaks with $\Delta_2 = 10$. The respective values of the model signal are: 54.4, 51.1, 60.7, 49.6, 54.8 and 64.7.

An exemplary calculation of the model signal involves iteratively decreasing values of $\Delta_1$ and increases the values of $\Delta_2$, i.e. this gives a sequence of $(k, \Delta_k) = (1,-1), (2, 1), (1,-2), (2, 2), ...$ etc. After a limited number of iterations, obviously two indices will coincide and the computation of the reference waveform can be stopped:

$$\exists_{j:0 \leq j < N_p - 1} : \left[(\underline{p})_{j+1} + \Delta_1 - 1\right] \leq \left[(\underline{p})_j + \Delta_2 + 1\right].$$

In this non-limiting example, the stopping occurs when:

$$(\Delta_2 - \Delta_1 + 2) \geq \min_{0 < i < (N_p - 1)} \left[(\underline{p})_{j+1} - (\underline{p})_j\right]$$

The term $\lfloor (\underline{p})_{j+1} - (\underline{p})_j \rfloor$ essentially gives the interval between two neighboring peaks. The values of $\Delta_k$ with k = 1, 2 at the moment that the condition of the previous equation occurs will be denoted by $\Delta_k^*$ with $k = 1, 2$. These values denote the offsets (toward the left and right) that represent the boundaries of the model for each wave (centered around the occurrences of peak-detections). For the given example, the values of $\Delta_1$ and $\Delta_2$ are respectively -36 and 36. The dashed curve in Fig. 11B represents the model signal 18e after the stopping criterion.

Alternatively, a different stopping criterion can be employed for left and/or right side. For example, the determination of values of the model signal towards the left-side ($\Delta_1 < 0$) ends earlier than toward the right-side ($\Delta_2 > 0$). For example, the stopping criterion to the left-side can be defined as:

$$\forall_j : (\underline{x}_p)_j \cdot (1 - \psi \, \Delta_1) < 0 \, .$$

The two stopping criteria described above can also be combined or alternative stopping criteria can be used.

After the aforementioned determination of values of the model signal $\underline{m}$, there can still be unmodelled values in $m$. In other words, not all values of $m$ are filled. This can also be seen in Fig. 11B, where the values of the reference signal 18e between samples 210 and 240 remain unmodelled and the model signal 18e shows a gap.

The unmodeled values in-between the peak-detections of the segment can be filled by means of, for example, linear interpolation. For each pair subsequent peak-detections ($N_p$ - 1 pairs), a vector $\underline{g}$ having $N_p$ - 1 elements can be computed. The elements of the vector indicate the lengths of the respective gaps in samples. For the i'th pair the i'th element of the vector can be computed by

$$\forall_{0 \le i < (N_p - 1)} : (\underline{g}_i)_i = (\underline{p})_{i+1} - (\underline{p})_i + \Delta_1^* - \Delta_2^* + 1,$$

where $\Delta_1^*$ and $\Delta_2^*$ are defined as being the offsets (toward the left and right respectively) that represent the samples at boundaries of the model signal for each scaled average waveform (centered around the peak-detections). The elements in the vector $\underline{g}$ for the example shown in Fig. 11B are: 1, 2, 51, 5 and 5.

The gaps in the model $\underline{m}$ are then filled up by computing:

$$\forall_{0 \le j < (N_p - 1)}, \forall_{1 \le i < (\underline{g}_j) - 1} :$$

$$(m)_{[(\underline{p})_j + \Delta_2 + i]} = (m)_{[(\underline{p})_j + \Delta_2]} + \frac{(m)_{[(p)_j + \Delta_2 + \underline{g}_j]} - (m)_{[(p)_j + \Delta_2]}}{i}$$

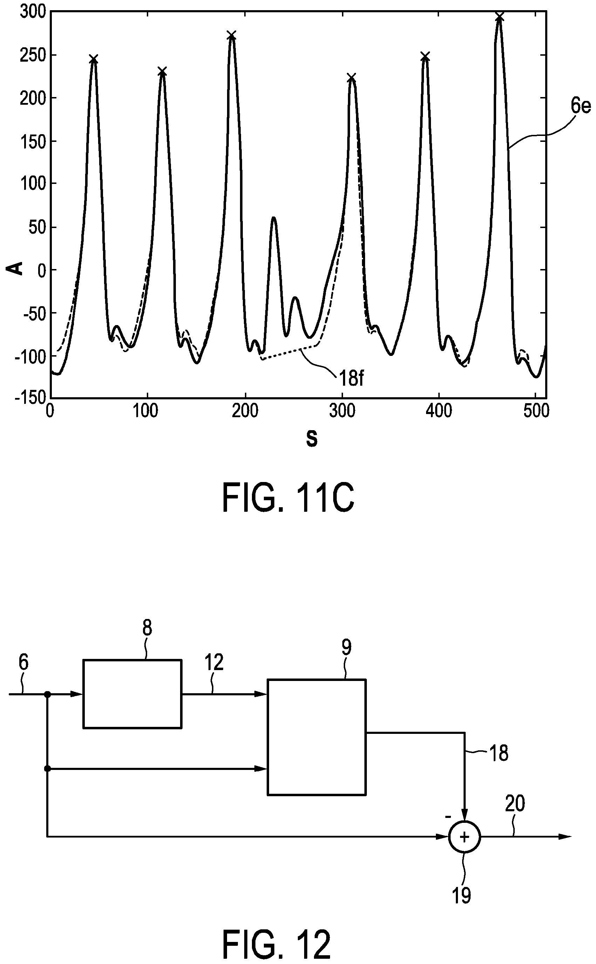

Fig. 11C shows the result of the interpolation step as a dashed curve 18f.

Optionally, a quality metric descriptive of a quality of the feature signal can be determined by computing a normalized magnitude difference between the original data $\underline{x}$ and the model $\underline{m}$. Since the model signal may not have been determined for all samples of the physiological signal, the calculation of the quality metric may be limited to those values where the model signal exists. For example if samples at a boundary of the segment have not been modeled, the determination of the quality metric may be limited to the range between a leftmost sample and a rightmost sample of the model signal, hence:

$$\text{quality metric} = \frac{\sum\limits_{i=(\underline{p})_0 + \Delta_1^*}^{(\underline{p})_{Np-1} + \Delta_2^*} \left| (\underline{x})_i - (\underline{m})_i \right|}{\sum\limits_{i=(\underline{p})_0 + \Delta_1^*}^{(\underline{p})_{Np-1} + \Delta_2^*} \left| (\underline{x})_i \right|} \, .$$

The quality metric as calculated above can be directly used for indicating a quality of the feature signal or can be used in a classifier for evaluating the physiological signal. For example, a classifier-tree can be used:

```
label = 0;
if (energy of the waveform < bound1),
        if ( peak-to-peak variance < bound2 OR quality metric < bound3),
         label = 1;
         end
```

```
end
```

Advantageously, the energy of the physiological signal should be below a certain bound (bound1) in order to avoid classifications in case of heavy movements measured by the accelerometer, e.g. periodic walking signals.

The peak-to-peak variance can be used for the classification in case of regular signals, i.e. signals without arrhythmia, whereas the classifier according to an aspect of the present invention can be used in case of arrhythmias.

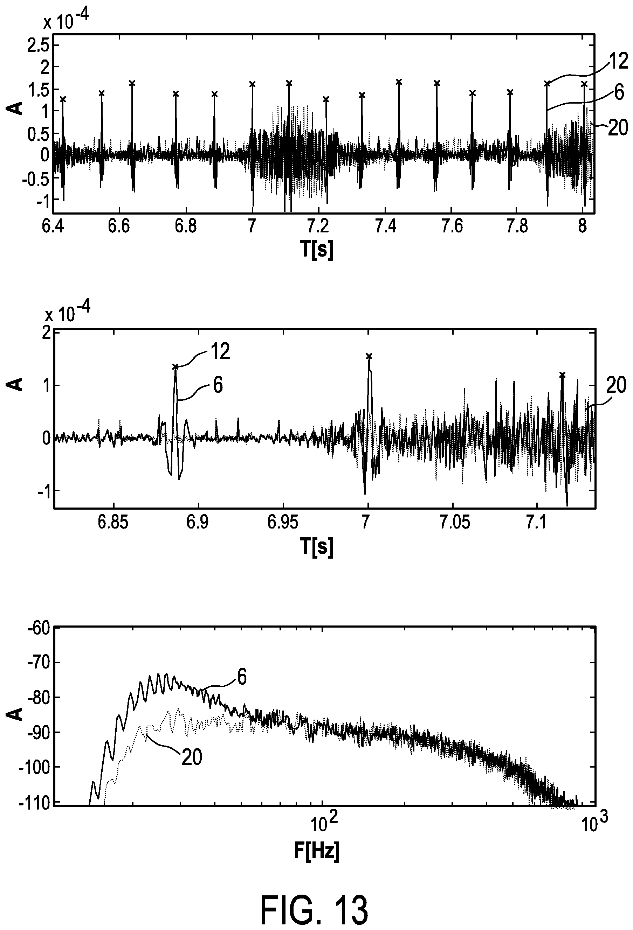

A further advantageous application of the processing apparatus 9 according to an aspect of the present invention is described with reference to Figs. 12 and 13.

Fig. 12 shows a system for removing an electrocardiogram (ECG) overlay in an electromyogram (EMG) measurement. An EMG represents a muscle activity, for example a parasternal muscle activity which is related to the breathing effort. Hence, parameter can be extracted from an EMG signal, that is indicative of a respiratory acticity, in particular indicative of how much difficulty a person has to perform the breathing. For example, when the EMG is measured at the 2nd intercostal space on the chest of a person, also known as parasternal EMG measurement, the EMG signal is related to the amount of activation of the parasternal muscles. However, the measured physiological signal 6 comprises both the desired EMG signal due to parasternal muscle activity as well as an ECG signal due to cardiac activity. The ECG signal can be dominant over the EMG signal.

The system shown in Fig. 12 comprises a feature detector 8, the processing apparatus 9 according to an aspect of the present invention, and an adder 19. The feature detector 8 receives the measured physiological signal 6 at an input and provides a feature signal 12 descriptive of occurrences of a feature in the physiological signal 6 at an output. In this embodiment, the feature detector 8 is configured as a peak detector that is configured to perform a QRS peak-detection to locate the R peaks of an ECG wave. For example, the peak detector uses the Pan-Tomkins algorithm (J.Pan, W.J. Tomkins, "A real-time QRS detection algorithm", IEEE Trans. Biomed. Eng. 1985, vol. 32, pp. 230-236).

The processing apparatus 9 obtains the physiological signal 6, containing at least two wave cycles originating from cardiac activity (ECG signal) and respiration (EMG signal), and the feature signal 12 from the feature detector 8. The model signal 18 is provided at an output of the processing apparatus 9. In this embodiment, the feature signal 12 indicates occurrences of cardiac pulses. Therefore, the resulting model signal 18 comprises instances of the average waveform of the physiological signal 6 at the occurrences of the cardiac pules in the physiological signal. Thus, the model signal 18 represents a model of the ECG signal.

The model signal 18 can be subtracted from the physiological signal 6 with the adder 19, wherein the model signal is provided at an inverted input of the adder 19. The output signal is a difference signal or residual signal 20 which is effectively free from the ECG signal. The difference signal can be evaluated, for example to determine the respiration rate or the respiratory effort. The model signal 18 can also be used for further processing or for example be presented to the subject or physician as a clean ECG signal.

Fig. 13 shows the measured physiological signal 6, with a dominant contribution of the ECG signal, the feature signal 12, indicated by crosses, and the ECG-removed residual signal 20. The horizontal axis in the upper and middle graph denotes the time T in seconds, whereas the vertical axis represents the amplitude. The middle graph shows a magnification of a segment from the upper graph. The lower graph shows a spectrum of the physiological signal 6 and the residual 20.

The ECG waves due to cardiac pulses are cancelled in the residual signal 20. This can also be seen from the spectrum shown in the lower plot of Fig. 13, where a power of the residual signal 20 has been reduced by about 15-20 dB compared to the physiological signal 6. An advantage of this signal processing according to an aspect of the present invention is the fact that the EMG signal remains untouched by the ECG removal technique (Fig. 13 upper and middle graph). This in contrast with conventional filtering techniques (like low-pass filtering or median filtering or Savitsky-Golay filtering) where the EMG waveform is modified by the filtering process. While the capability of distinguishing different contributions in a physiological signal has been illustrated for an ECG/EMG signal, it should be noted that such illustration is to be considered exemplary and not restrictive.

In summary, the present invention proposes a processing apparatus, processing method and device for processing a physiological signal, based on an obtained physiological signal and an obtained feature signal descriptive of occurrences of a feature in the physiological signal, by determining an average waveform of the physiological signal around said occurrences of the feature in the physiological signal and by determining a model signal comprising amplitude-scaled instances of the average waveform of the physiological signal at the occurrences of the feature in the physiological signal. Thus, the proposed processing apparatus provides a model signal which can, for example, be used for determining a quality of the feature signal (which forms the basis for determining a vital sign of the subject) even in case of arrhythmias and also in case of fluctuating amplitudes. Furthermore, the model signal can serve for distinguishing different contributions to a physiological signal.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

A computer program may be stored/distributed on a suitable medium, such as an optical storage medium or a solid-state medium supplied together with or as part of other hardware, but may also be distributed in other forms, such as via the Internet or other wired or wireless telecommunication systems.

**Claims**

1. A processing apparatus (9) for processing a physiological signal (6) configured to perform the steps of:

   - obtaining the physiological signal (6) containing at least two wave cycles originating from a physiological process,
   - obtaining a feature signal (12) descriptive of occurrences of a feature (P) in the physiological signal (6), and wherein the feature (P) comprises a predetermined energy, slope, inflection points, local extrema or combinations thereof,
   - determining an average waveform of the physiological signal (6) around said occurrences of the feature (P) in the physiological signal (6), and
   - determining a model signal (18) comprising amplitude-scaled instances of the average waveform of the physiological signal (6) placed at the occurrences of the feature (P) in the physiological signal (6),

   wherein the step of determining the average waveform comprises the step of determining an average relative change between a value of the physiological signal (6) at occurrences of the feature (P) and a value of a neighboring sample of the physiological signal (6).

2. The processing apparatus according to claim 1, wherein the physiological signal (6) is a movement signal descriptive of a physiological movement.

3. The processing apparatus according to claim 1, wherein the feature (P) of the physiological signal is a peak of the physiological signal.

4. The processing apparatus according to claim 1, further configured to perform the step of comparing the model signal (18) with the physiological signal (6).

5. The processing apparatus according to claim 4, wherein the comparing step comprises the computation of a difference signal between the model signal (18) and the physiological signal (6).

6. The processing apparatus according to claim 5, wherein the comparing step further comprises the computation of an energy of the difference signal descriptive of the quality of the feature signal (12) or the physiological signal (6).

7. The processing apparatus according to claim 1, wherein step of determining the average waveform comprises repeating the step of determining an average relative change for different neighboring samples preceding and/or succeeding occurrences of the feature (P).

8. The processing apparatus according to claim 7, wherein said repeating is performed until reaching a stop criterion, in particular until a relative change has been determined for all samples of the physiological signal between at least one pair of succeeding occurrences of the features (P) having the smallest temporal separation.

9. The processing apparatus according to claim 1, wherein the step of determining the model signal (18) further comprises the step of interpolating the model signal (18) between instances of the average waveform.

10. The processing apparatus according to claim 1, wherein the processing apparatus is further configured to evaluate a variation of a temporal separation of occurrences of a feature (P) in the feature signal (12).

**11.** The processing apparatus according to claim 1,
wherein the processing apparatus is further configured to evaluate the physiological signal (6) and/or feature signal (12) in time-domain.

**12.** A processing method for processing a physiological signal (6) with a processing apparatus, the processing method comprising the steps of:

- obtaining the physiological signal (S11) containing at least two wave cycles originating from a physiological process,
- obtaining a feature signal (12) descriptive of occurrences of a feature (P) of the physiological signal (S12), and wherein the feature (P) comprises a predetermined energy, slope, inflection points, local extrema or combinations thereof,
- determining an average waveform of the physiological signal (6) around said occurrences of the feature (P) of the physiological signal (6) (S 13),
- determining a model signal (18) comprising amplitude-scaled instances of the average waveform of the physiological signal (6) placed at the occurrences of the feature (P) of the physiological signal (6) (S14),

wherein the step of determining the average waveform comprises the step of determining an average relative change between a value of the physiological signal (6) at occurrences of the feature (P) and a value of a neighboring sample of the physiological signal (6).

**13.** Computer program comprising program code means for causing a computer to carry out the steps of the method as claimed in claim 12 when said computer program is carried out on the computer.

**14.** A system (1) for determining a vital sign of a subject comprising

- a sensor (5) for measuring a physiological signal (6), and
- a processing unit (7) comprising a first processing apparatus (8) for determining a feature signal (12) descriptive of occurrences of a feature (P) of the physiological signal (6), and a second processing apparatus (9) for processing a physiological signal (6), wherein the second processing apparatus (9) is a processing apparatus according to any of claims 1-11 configured to determine a model signal (18),

wherein the processing unit (7) is further configured to determine the vital sign of the subject based on the physiological signal (6) taking into account the model signal (18).

**Patentansprüche**

**1.** Verarbeitungsapparat (9) zur Verarbeitung eines physiologischen Signals (6)
konfiguriert, zur Ausführung folgender Schritte:

- Erhalten des physiologischen Signals (6), das mindestens zwei Wellenzyklen enthält, die von einem physiologischen Prozess stammen,
- Erhalten eines Merkmalssignals (12), das das Auftreten eines Merkmals (P) in dem physiologischen Signal (6) beschreibt, und wobei das Merkmal (P) eine vorbestimmte Energie, Steigung, Wendepunkte, lokale Extrema oder Kombinationen davon umfasst,
- Bestimmen einer durchschnittlichen Wellenform des physiologischen Signals (6) um das Auftreten des Merkmals (P) in dem physiologischen Signal (6), und
- Bestimmen eines Modellsignals (18), das amplitudenskalierte Instanzen der durchschnittlichen Wellenform des physiologischen Signals (6) umfasst, die an den Auftreten des Merkmals (P) in dem physiologischen Signal (6) platziert sind, wobei der Schritt des Bestimmens der durchschnittlichen Wellenform den Schritt des Bestimmens einer durchschnittlichen relativen Änderung zwischen einem Wert des physiologischen Signals (6) an Auftreten des Merkmals (P) und einem Wert einer benachbarten Probe des physiologischen Signals (6) umfasst.

**2.** Verarbeitungsapparat nach Anspruch 1, wobei das physiologische Signal (6) ein Bewegungssignal ist, das eine physiologische Bewegung beschreibt.

**3.** Verarbeitungsapparat nach Anspruch 1, wobei das Merkmal (P) des physiologischen Signals eine Spitze des physiologischen Signals ist.

4. Verarbeitungsapparat nach Anspruch 1, der ferner so konfiguriert ist, dass er den Schritt des Vergleichs des Modellsignals (18) mit dem physiologischen Signal (6) durchführt.

5. Verarbeitungsapparat nach Anspruch 4, wobei der Vergleichsschritt die Berechnung eines Differenzsignals zwischen dem Modellsignal (18) und dem physiologischen Signal (6) umfasst.

6. Verarbeitungsapparat nach Anspruch 5, wobei der Vergleichsschritt ferner die Berechnung einer Energie des Differenzsignals umfasst, die die Qualität des Merkmalssignals (12) oder des physiologischen Signals (6) beschreibt.

7. Verarbeitungsapparat nach Anspruch 1, wobei der Schritt des Bestimmens der durchschnittlichen Wellenform das Wiederholen des Schritts des Bestimmens einer durchschnittlichen relativen Änderung für verschiedene benachbarte Abtastwerte vor und/oder nach dem Auftreten des Merkmals (P) umfasst.

8. Verarbeitungsapparat nach Anspruch 7, wobei die Wiederholung bis zum Erreichen eines Stoppkriteriums durchgeführt wird, insbesondere bis eine relative Änderung für alle Abtastwerte des physiologischen Signals zwischen mindestens einem Paar von aufeinanderfolgenden Auftritten der Merkmale (P) mit dem kleinsten zeitlichen Abstand bestimmt worden ist.

9. Verarbeitungsapparat nach Anspruch 1, wobei der Schritt des Bestimmens des Modellsignals (18) weiterhin den Schritt des Interpolierens des Modellsignals (18) zwischen Instanzen der durchschnittlichen Wellenform umfasst.

10. Verarbeitungsapparat nach Anspruch 1, wobei der Verarbeitungsapparat ferner so konfiguriert ist, dass er eine Variation einer zeitlichen Trennung des Auftretens eines Merkmals (P) im Merkmalssignal (12) auswertet.

11. Verarbeitungsapparat nach Anspruch 1, wobei der Verarbeitungsapparat des Weiteren konfiguriert ist, das physiologische Signal (6) und/oder das Merkmalssignal (12) im Zeitbereich auszuwerten.

12. Verarbeitungsverfahren zur Verarbeitung eines physiologischen Signals (6) mit einem Verarbeitungsapparat, wobei das Verarbeitungsverfahren die folgenden Schritte umfasst:

- Erhalten des physiologischen Signals (S11), das mindestens zwei Wellenzyklen enthält, die von einem physiologischen Prozess stammen,
- Erhalten eines Merkmalssignals (12), das das Auftreten eines Merkmals (P) des physiologischen Signals (S12) beschreibt, und wobei das Merkmal (P) eine vorbestimmte Energie, Steigung, Wendepunkte, lokale Extrema oder Kombinationen davon umfasst,
- Bestimmen einer durchschnittlichen Wellenform des physiologischen Signals (6) um das Auftreten des Merkmals (P) des physiologischen Signals (6) (S13),
- Bestimmen eines Modellsignals (18), das amplitudenskalierte Instanzen der durchschnittlichen Wellenform des physiologischen Signals (6) umfasst, die an den Auftritten des Merkmals (P) des physiologischen Signals (6) platziert sind (S14), wobei der Schritt des Bestimmens der durchschnittlichen Wellenform den Schritt des Bestimmens einer durchschnittlichen relativen Änderung zwischen einem Wert des physiologischen Signals (6) an Auftritten des Merkmals (P) und einem Wert einer benachbarten Probe des physiologischen Signals (6) umfasst.

13. Computerprogramm mit Programmcodemitteln, um einen Computer zu veranlassen, die Schritte des Verfahrens nach Anspruch 12 auszuführen, wenn das Computerprogramm auf dem Computer ausgeführt wird.

14. System (1) zur Bestimmung eines Vitalzeichens eines Patienten mit

- einem Sensor (5) zur Messung eines physiologischen Signals (6), und
- eine Verarbeitungseinheit (7), die einen ersten Verarbeitungsapparat (8) zum Bestimmen eines Merkmalssignals (12), das das Auftreten eines Merkmals (P) des physiologischen Signals (6) beschreibt, und einen zweiten Verarbeitungsapparat (9) zum Verarbeiten eines physiologischen Signals (6) umfasst, wobei der zweite Verarbeitungsapparat (9) ein Verarbeitungsapparat nach einem der Ansprüche 1 bis 11 ist, der so konfiguriert ist, dass er ein Modellsignal (18) bestimmt, wobei die Verarbeitungseinheit (7) ferner so konfiguriert ist, dass sie das Vitalzeichen des Patienten auf der Grundlage des physiologischen Signals (6) unter Berücksichtigung des Modellsignals (18) bestimmt.

**Revendications**

1. Appareil de traitement (9) pour traiter un signal physiologique (6)
   configuré pour exécuter les étapes consistant à:

   - obtenir un signal physiologique (6) contenant au moins deux cycles d'ondes provenant d'un processus physiologique,
   - obtenir un signal de caractéristique (12) décrivant les occurrences d'une caractéristique (P) dans le signal physiologique (6), et dans lequel la caractéristique (P) comprend une énergie, une pente, des points d'inflexion, des extrema locaux ou des combinaisons de ceux-ci prédéterminés,
   - déterminer une forme d'onde moyenne du signal physiologique (6) autour desdites occurrences de la caractéristique (P) dans le signal physiologique (6), et
   - déterminer un signal modèle (18) comprenant des instances mises à l'échelle en amplitude de la forme d'onde moyenne du signal physiologique (6) placées aux occurrences de la caractéristique (P) dans le signal physiologique (6),

   dans lequel l'étape de détermination de la forme d'onde moyenne comprend l'étape de détermination d'un changement relatif moyen entre une valeur du signal physiologique (6) aux occurrences de la caractéristique (P) et une valeur d'un échantillon voisin du signal physiologique (6).

2. Appareil de traitement selon la revendication 1, dans lequel le signal physiologique (6) est un signal de mouvement descriptif d'un mouvement physiologique.

3. Appareil de traitement selon la revendication 1, dans lequel la caractéristique (P) du signal physiologique est un pic du signal physiologique.

4. Appareil de traitement selon la revendication 1, configuré en outre pour exécuter l'étape de comparaison du signal modèle (18) avec le signal physiologique (6).

5. Appareil de traitement selon la revendication 4, dans lequel l'étape de comparaison comprend le calcul d'un signal de différence entre le signal modèle (18) et le signal physiologique (6).

6. Appareil de traitement selon la revendication 5, dans lequel l'étape de comparaison comprend en outre le calcul d'une énergie du signal de différence descriptive de la qualité du signal de caractéristique (12) ou du signal physiologique (6).

7. Appareil de traitement selon la revendication 1, dans lequel l'étape de détermination de la forme d'onde moyenne comprend la répétition de l'étape de détermination d'un changement relatif moyen pour différents échantillons voisins précédant et/ou suivant les occurrences de la caractéristique (P).

8. Appareil de traitement selon la revendication 7, dans lequel ladite répétition est effectuée jusqu'à atteindre un critère d'arrêt, en particulier jusqu'à ce qu'un changement relatif ait été déterminé pour tous les échantillons du signal physiologique entre au moins une paire d'occurrences successives des caractéristiques (P) ayant la plus petite séparation temporelle.

9. Appareil de traitement selon la revendication 1, dans lequel l'étape de détermination du signal modèle (18) comprend en outre l'étape d'interpolation du signal modèle (18) entre des instances de la forme d'onde moyenne.

10. Appareil de traitement selon la revendication 1, dans lequel l'appareil de traitement est en outre configuré pour évaluer une variation d'une séparation temporelle d'occurrences d'une caractéristique (P) dans le signal de caractéristique (12).

11. Appareil de traitement selon la revendication 1, dans lequel l'appareil de traitement est en outre configuré pour évaluer le signal physiologique (6) et/ou le signal caractéristique (12) dans le domaine temporel.

**12.** Procédé de traitement pour traiter un signal physiologique (6) avec un appareil de traitement, le procédé de traitement comprenant les étapes consistant à:

- obtenir le signal physiologique (S11) contenant au moins deux cycles d'ondes provenant d'un processus physiologique,
- obtenir un signal de caractéristique (12) décrivant les occurrences d'une caractéristique (P) du signal physiologique (S12), et dans lequel la caractéristique (P) comprend une énergie, une pente, des points d'inflexion, des extrema locaux ou des combinaisons de ceux-ci prédéterminés,
- déterminer une forme d'onde moyenne du signal physiologique (6) autour desdites occurrences de la caractéristique (P) du signal physiologique (6) (S13),
- déterminer un signal modèle (18) comprenant des instances mises à l'échelle en amplitude de la forme d'onde moyenne du signal physiologique (6) placées aux occurrences de la caractéristique (P) du signal physiologique (6) (S14),

dans lequel l'étape de détermination de la forme d'onde moyenne comprend l'étape de détermination d'un changement relatif moyen entre une valeur du signal physiologique (6) aux occurrences de la caractéristique (P) et une valeur d'un échantillon voisin du signal physiologique (6).

**13.** Programme d'ordinateur comprenant des moyens de code de programme pour amener un ordinateur à exécuter les étapes du procédé selon la revendication 12 lorsque ledit programme d'ordinateur est exécuté sur l'ordinateur.

**14.** Système (1) pour déterminer un signe vital d'un sujet comprenant

- un capteur (5) pour mesurer un signal physiologique (6), et
- une unité de traitement (7) comprenant un premier appareil de traitement (8) pour déterminer un signal caractéristique (12) descriptif des occurrences d'une caractéristique (P) du signal physiologique (6), et un deuxième appareil de traitement (9) pour traiter un signal physiologique (6),

dans lequel le deuxième appareil de traitement (9) est un appareil de traitement selon l'une quelconque des revendications 1 à 11 configuré pour déterminer un signal modèle (18), dans lequel l'unité de traitement (7) est en outre configurée pour déterminer le signe vital du sujet sur la base du signal physiologique (6) en tenant compte du signal modèle (18).

**FIG. 1**

**FIG. 2**

**FIG. 3**

FIG. 4

FIG. 5

acc_x
acc_y
acc_z

13    6   8    15   9   14

6    12   16

# FIG. 6

S11
S12
S13
S14
S15
14

# FIG. 7

FIG. 8

FIG. 9A

FIG. 9B

FIG. 9C

FIG. 9D

FIG. 10A

FIG. 10B

FIG. 11A

FIG. 11B

FIG. 11C

FIG. 12

FIG. 13

**EP 3 048 972 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040039420 A1
- US 20060224074 A1
- US 20100312075 A1
- US 2005222510 A1

**Non-patent literature cited in the description**

- **J.PAN ; W.J. TOMKINS.** A real-time QRS detection algorithm. *IEEE Trans. Biomed. Eng.,* 1985, vol. 32, 230-236